# FASCICULE DE BREVET EUROPEEN

(11) **EP 1 307 182 B1**
(45) Date de publication et mention de la délivrance du brevet: **30.03.2005**
(21) Numéro de dépôt: 01958205.5
(22) Date de dépôt: 31.07.2001
(51) Int. Cl.: A61K 9/70, A61K 47/10, A61K 47/14

(54) **COMPOSITION PHARMACEUTIQUE POUR ADMINISTRATION TRANSDERMIQUE DE BEFLOXATONE**
PHARMAZEUTISCHE ZUSAMMENSETZUNG ZUR VERABREICHUNG VON BEFLOTAXONE
PHARMACEUTICAL COMPOSITION FOR TRANSDERMAL DELIVERY OF BEFLOXATONE

(30) Priorité: 01.08.2000 FR 0010135
(43) Date de publication de la demande: 07.05.2003
(73) Titulaire: Sanofi-Aventis, 75013 Paris (FR)
(72) Inventeur: CHARLIER, Anne, F-75007 Paris (FR); HENRION, Louis, F-75005 Paris (FR)
(74) Mandataire: Weber, Mathieu
(86) Numéro de dépôt international: PCT/FR2001/002494
(87) Numéro de publication internationale: WO 2002/009704

(56) Documents cités:
- WO-A-01/12176
- FR-A- 2 768 338

## Description

La présente invention a pour objet une composition pharmaceutique pour administration transdermique de béfloxatone.

Plus spécifiquement, la présente invention est relative à une composition pharmaceutique transdermique formulée de façon à permettre l'absorption de la béfloxatone au travers de la peau en un site d'application choisi.

Au titre de la présente invention on entend par béfloxatone la 3-[4-(4,4,4-trifluoro-3(*R*)-ydroxybutoxy)phenyl]5(*R*)-methoxymethyl-2-oxazolidinone, qui est connue pour son activité d'antidépresseur. Il s'agit d'un inhibiteur réversible de la MAO-A comportant à la fois une très haute affinité pour l'isoforme A (MAO-A) et une bonne sélectivité versus l'isoforme B (MAO-B), et qui n'affecte pas la recapture de la noradrénaline (NA), sérotonine (5-HT) ou dopamine (DA).

Sa synthèse chimique est décrite dans EP 424244.

FR-A-décrit une composition pharmaceutique pour administration orale ou parentérale de béfloxatone et WO-A-01 12176 décrit une composition pharmaceutique pour administration orale de béfloxatone.

Le but de la présente invention est d'obtenir une libération contrôlée du principe actif afin d'éviter des prises quotidiennes répétées de principe actif lorsqu'il est administré par voie orale de façon classique. La compliance du traitement est ainsi nettement améliorée.

La présente invention est relative à une composition pharmaceutique pour administration transdermique de béfloxatone, destinée notamment au sevrage tabagique, au traitement des états dépressifs ou de l'obésité.
Un autre but de la présente invention est de permettre une absorption rapide de la béfloxatone au site d'application avec un taux de béfloxatone suffisant pour atteindre l'effet thérapeutique recherché.

Dans le cadre de la présente invention, les quantités, sauf indication contraire, sont exprimées en % en poids de la composition totale.

Plus spécifiquement, l'invention consiste en une composition pharmaceutique caractérisée en ce qu'elle comprend un principe actif consistant en la béfloxatone, ainsi qu'au moins un promoteur d'absorption, ladite composition étant formulée pour permettre une administration transcutanée.
La composition pharmaceutique peut prendre la forme de gel, de pommade ou d'émulsion pour une application locale, d'un timbre ou patch transdermique ou d'un film déposé par un spray.

Dans le cas du gel ou de la pommade, d'autres excipients peuvent également être ajoutés dans les compositions selon la présente invention. On peut ainsi citer des excipients classiques tels des parfums, des huiles essentielles, des conservateurs, des agents hydratants apaisants, ou des colorants.

Le mode d'application préféré est un film transdermique, en particulier au moyen d'un timbre (patch) ou vaporisation d'un spray qui, par évaporation de solvant, laisse un film sur la peau. Un film transdermique permet une administration lente et régulière du principe actif. L'autonomie du patient vis-à-vis de son traitement est ainsi favorisée. Le patch permet par exemple d'obtenir une libération de la composition qui peut durer entre 8 et 72 heures.

Ainsi, plus particulièrement, chaque composition entrant dans la préparation de ce film transdermique comprend, outre le polymère, un à trois promoteurs d'absorption tels que définis ci-dessous (systèmes simples, binaires ou ternaires tels que décrits dans les exemples).

Ainsi, on entend par le terme promoteur d'absorption, un composé pharmaceutiquement acceptable, qui permet d'améliorer le passage du principe actif à travers la peau. A cette fin, le promoteur d'absorption peut, notamment, modifier la perméabilité ou changer l'état de la surface de la peau afin de faciliter le passage.
Les promoteurs d'absorption peuvent appartenir aux différentes catégories suivantes : (i) les alcools comprenant de 2 à 36 atomes de carbone, estérifiés ou non par des acides organiques comprenant de 1 à 6 atomes de carbone, (ii) les acides gras comprenant de 5 à 30 atomes de carbone, estérifiés ou non par des alcools comprenant de 1 à 6 atomes de carbone, (iii) les sels alcalins et alcaline-terreux d'acides gras comprenant de 5 à 30 atomes de carbone.

Parmi les alcools, peuvent être particulièrement cités l'éthanol, l'isopropanol et les alcools gras de formule R₁OH où R₁ représente un radical alkyle linéaire ou ramifié, saturé ou insaturé, et comprenant 6 à 30 atomes de carbone. D'autres alcools tels que l'alcool benzylique, le propylène glycol peuvent être utilisés comme promoteurs d'absorption.

A titre d'acides gras pouvant être utilisés comme promoteurs d'absorption, peuvent être cités ceux de formule R₂COOH où R₂ représente un radical alkyle linéaire ou ramifié, saturé ou insaturé, et comprenant 6 à 30 atomes de carbone. Ces acides gras peuvent former des esters avec les alcools méthyliques et éthyliques par exemple, ou avec le glycérol sous forme de mono, di ou triesters.
On préfère parmi les acides gras promoteurs d'absorption les acides caprylique, caprique, laurique stéarique et oléique.
De même, parmi les acides gras estérifiés on préférera le myristate d'isopropyle ou le propylene glycol monolaurate (Lauroglycol®).

Les sels alcalins et alcalino-terreux d'acides gras comprenant de 5 à 30 atomes de carbone pouvant être cités sont les sels de sodium, de potassium ou de calcium des acides gras mentionnés précédemment.

D'autres promoteurs d'absorption peuvent également être cités, parmi lesquels les tensioactifs et les dioxolanes.

Selon un mode de réalisation particulier de l'invention, lorsque le film transdermique est appliqué à l'aide d'un patch transdermique, ce dernier peut être constitué par un système matriciel, un système réservoir ou un système constitué d'enductions successives. Le dispositif transdermique peut en outre inclure des constituants propres à réaliser le système, à assurer sa conservation et à permettre son utilisation.
Dans le cas d'un système matriciel, ces constituants peuvent être scindés en trois groupes : supports passifs, constituants actifs du système, adhésifs. Le support passif peut être un film métallique, par exemple d'aluminium, un tissu ou un réseau non tissé de fibres naturelles ou artificielles, un film polymérique tel que polyéthylène, polypropylène, polytétrafluoro-éthylène, polymère cellulosique, acrylique, vinylique, silicone, acrylonitrile etc... Les constituants actifs du système peuvent être des films ou des matrices polymériques tels que : polyéthylène, polypropylène, polytétrafluoro-éthylène, polymère cellulosique, acrylique, vinylique, silicone, acrylonitriles etc...Le ou les adhésifs peuvent être constitués de caoutchouc naturel ou synthétique, polyisobutylène, polyacrylates, polyvinyléthers etc...
Les dispositifs transdermiques préférés dans le cadre de la présente invention sont ceux qui comportent une matrice polymérique telle que celles décrites ci-dessus et encore plus particulièrement ceux pour lesquels le polymère constituant le système matriciel est également adhésif, à l'exemple des polyacrylates. La composition dans le cas d'un timbre transdermique comprenant une matrice polymérique est caractérisée en ce qu'elle comprend (i) la béfloxatone, (ii) un à trois promoteurs d'absorption et (iii) un polymère.
Selon la présente invention, on peut obtenir timbres ou patch transdermiques selon des procédés conventionnels pour l'homme du métier.

Selon un autre mode particulier de réalisation de l'invention, lorsque le film transdermique est appliqué à l'aide d'un spray, ce dernier peut se présenter sous forme d'une solution. Elle se caractérise par le fait qu'elle comprend, outre (i) la béfloxatone, (ii) un à trois promoteurs d'absorption, (iii) un polymère ou copolymère formant un film souple. après évaporation du solvant et (iv) un solvant capable de dissoudre les constituants (i), (ii) et (iii) précédents et de s'évaporer facilement.
La composition se prépare de façon conventionnelle en mélangeant les constituants.

Parmi les polymères ou copolymères capables de former un film souple après évaporation, on retient plus particulièrement des polymères ou copolymères cellulosiques notamment parce qu'ils présentent après séchage une résistance à l'abrasion et une stabilité mécanique appropriées. Pour cette raison, des matrices cellulosiques de ce type pourront être rincées à l'eau sans crainte de détérioration
ou encore d'élimination du principe actif.
A titre d'exemple de tels polymères ou copolymères cellulosiques utilisables dans les solutions pour spray de l'invention, on peut citer l'éthylcellulose, l'acétate butyrate de cellulose, l'acétate propionate de cellulose ou une hydroxypropylméthylcellulose greffée ou non telle que l'hydroxypropylméthylcellulose acétate succinnate.
L'éthylcellulose représente le polymère cellulosique préféré et, en conséquence, la matrice polymérique de choix pour la formation d'un film souple au contact de la peau.
En outre, la matrice polymérique peut être constituée d'un copolymère vinylpyrrolidone / acétate de vinyle tel que le polyvinylpyrrolidone / acétate de vinyle.

La plupart des promoteurs d'absorption d'un principe actif étant généralement un solvant de celui-ci, le terme solvant est ici, sauf indication contraire, réservé au solvant du polymère, en particulier dans le cas présent où la formulation est un spray film. Le dit solvant est alors dans ce cas aussi bien solvant du polymère que du principe actif.
Ainsi les solvants peuvent être des solvants organiques tels que l'éthanol, l'isopropanol, l'acétate d'éthyle, l'acétone, le diéthyléther, ou encore un mélange de ces derniers.
Parmi ces solvants volatiles, l'éthanol qui est particulièrement acceptable physiologiquement, pourra être préférentiellement utilisé dans une formulation d'un spray film.

Une composition pharmaceutique selon l'invention, quelle que soit la forme qu'elle prend notamment patch transdermique ou solution pour spray, peut comprendre de 1 à 20% de béfloxatone.
La teneur en promoteur d'absorption par rapport à la quantité totale en principe actif peut varier de 5 à 40%.
La teneur en solvant dans la solution pour spray peut varier en fonction, notamment, de la nature et de la teneur en polymère le cas échéant, mais aussi de la nature et de la teneur en promoteur d'absorption. En fonction de ces différents paramètres, l'homme du métier pourra déterminer la teneur en solvant requise.
Exemple 1 : mesures de flux

### Mode opératoire

Un modèle *ex vivo* utilisant de la peau humaine prélevée lors d'opérations de chirurgie plastique (plasties mammaires et abdominales) et soigneusement conservée pour garantir le maintien de ses propriétés physiologiques a été utilisé pour tester les compositions et leur efficacité dans l'amélioration du passage transdermique. Ce modèle a été décrit initialement par T.J. Franz (Current Problems in Dermatology, 7,58-68, 1978) et par H. Durrheim et coll. (Journal of Pharmaceutical Sciences, 69,7,781-786, 9980).
Le *stratum corneum,* l'épiderme et une partie du derme sont excisés à l'aide d'un dermatome à une épaisseur de 250 µm. L'étude du passage transdermique est réalisée en cellules de diffusion. Ces cellules comportent deux compartiments (donneur et récepteur). Le compartiment récepteur est soumis à une agitation magnétique à 600 rpm. La surface d'échange entre les deux compartiments est de 0,636 cm². L'ensemble est maintenu à 32°C (température de la surface de la peau chez l'Homme) par circulation d'eau thermostatée autour des compartiments.
Des morceaux de peau dermatomée découpés en carrés de 1,5 à 2 cm de côté sont tendus entre les deux compartiments de la cellule de diffusion. La face *stratum corneum* est placée côté donneur et la face épidermique côté récepteur.
Le compartiment donneur contient la solution de béfloxatone saturée à 32°C à tester. Le compartiment récepteur contient une solution de NaCl 9 ‰ (sérum physiologique).
L'expérience est réalisée pendant 24 à 48 heures. Pendant les premières heures, les prélèvements sont effectués de manière rapprochée dans le temps afin de déterminer le temps de latence de la diffusion.
Les échantillons prélevés conservés à 32°C sont dosés par CLHP.
Le flux transcutané à l'équilibre est déterminé par la pente de la section linéaire de la courbe de diffusion correspondant à la quantité de béfloxatone ayant diffusé en fonction du temps.

### Résultats

Les résultats sont rassemblés dans les tableaux 1 à 4.

Ils portent sur la mesure du passage de la béfloxatone solubilisée dans des mélanges de promoteurs et /ou solvants pouvant rentrer soit dans la composition de patch, soit dans la composition de spray.
- En colonne "lag-time" figure le temps séparant le début de la mesure et le moment
où la diffusion devient réellement linéaire.
- En colonne "flux" figure la pente calculée de la section linéaire de la diffusion, C'est le flux transcutané.
- En colonne "concentration" figure la concentration initiale de béfloxatone dans le milieu donneur.

| **1. FLUX OBTENUS EN SOLUTION DANS DES SYSTÈMES SIMPLES** | | | |
|---|---|---|---|
| **Composition** | **Lag- time (h)** | **Flux (µg/cm**^{**2**}**/h) c.v. (%)** | **Concentration (mg/mL)** |
| Propylène glycol | -9,1 ± 16.3 | 2,41 ± 1,69 70 % n = 4 | 57,4 - 60,3 |
| Isopropyle myristate | 2,4 ± 0,7 | 3,57 ± 1,09 31 % n = 3 | 2,8-3,1 |
| Glycéride C₈-C₁₀ éthoxylé Labrafac® hydrophile (Gatefossé) | 5,3 ± 9,2 | 7,64 ± 7,29 95 % n = 4 | 36,6 - 43,5 |
| Propylène glycol monolaurate Lauroglycol® (Gattefossé) | 2,0 ± 1,1 | 19,82 ± 2,63 13 % n = 4 | 23,2 - 25,7 |

| **2. FLUX OBTENUS EN SOLUTION DANS DES SYSTÈMES BINAIRES** | | | |
|---|---|---|---|
| **Composition** | **Lag- time (h)** | **Flux (µg/cm**^{**2**}**/h) c.v. (%)** | **Concentration (mg/mL)** |
| Isopropyle myristate/acide caprylique 90/10 | 10,6 ± 5,9 | 6,02 ± 0,41 7 % n=2 | 3,2 |
| Lauroglycol®/acide oléique 90/10 | 2,3 ± 1,2 | 18,91 ± 1,66 9%n=3 | 19,7 - 23,3 |
| Lauroglycol®/acide linoléique 90110 | 2,9 ± 0,9 | 19,39 ± 4,87 25 % n=4 | 20,4 - 24,3 |
| Lauroglycol®/acide caprylique 90/10 | 4,6 ± 0,2 | 21,14 ± 4,28 18 % n=3 | 21,4 - 21,7 |

| **3. FLUX OBTENUS EN SOLUTION DANS DES SYSTÈMES TERNAIRES** | | | |
|---|---|---|---|
| **Composition** | **Lag-time (h)** | **Flux (µg/cm**^{**2**}**/h) c.v. (%)** | **Concentration (mg/mL)** |
| Lauroglycol®/ipm/acide caprylique 80/10/10 | 6,2 ± 2,2 | 17,19 ± 4,67 27 % n=4 | 16,0 - 17,0 |
| Lauroglycol®/pg/acide caprylique 80/10/10 | 4,2 ± 1,2 | 39,41 ± 8,80 22 % n=4 | 25,1 - 28,0 |
| Lauroglycol®/pg/acide caprylique 45/45/10 | 13,4 ± 4,4 | 40,35 ± 14,69 36 % n=4 | 49,6 - 52,5 |

| **4.FLUX OBTENUS EN SOLUTION EN PRESENCE D'ETHANOL** | | | |
|---|---|---|---|
| **Composition** | **Lag- time (h)** | **Flux (µg/cm**^{**2**}**/h) c.v. (%)** | **Concentration (mg/mL)** |
| Ethanol | 10,5 ± 1,3 | 5,0 ± 3,5 70%n=4 | 209,2 - 271,3 |
| Ethanol/acide Caprylique 90/10 | 0,2 ± 2,6 | 2,78 ± 2,25 81 % n=3 | 216,2 |
| Ethanol/acide Oléique 90/10 | 11,5 ± 1,7 | 4,5 ± 3,0 67 % n=2 | 196,8 |
| Lauroglycol®/éthanol 50/50 | 6,3 ± 6,7 | 14,17 ± 3,16 22 % n=3 | 137,9 |
| Lauroglycol®/éthanol 90/10 | 2,9 ± 0,4 | 42,03 ± 5,29 13 % n=3 | - |
| Lauroglycol®/éthanol/acide caprylique 45/45/10 | 14,3 ± 2,8 | 9,76 ± 9,87 101 % n=4 | 102,6 -107,1 |
| Lauroglycol®/éthanol/acide caprylique 80/10/10 | 13,4 ± 6,9 | 47,29 ± 12,20 26 % n=4 | 29,2 - 37,5 |

### Exemple 2 : formulation d'un patch

On effectue un mélange pondéral 80:10:10 des promoteurs Lauroglycol®, propylène glycol et acide caprylique. On introduit 15 % du mélange promoteur dans le Duro-Tak® 387-2353 non réticulé, polymère autoadhésif qui appartient à la famille des polyacrylates. On ajoute de la béfloxatone en proportion telle que le film matriciel actif obtenu soit dosé à 15 mg/25 cm². On enduit sur un backing Scotchpak® 1009 (support constitué d'une feuille d'aluminium enduite de polyéthylène servant de support extérieur- souvent transparente ou de couleur chair) à la vitesse de 6 cm/s sur une table à vide avec un filmographe réglé à 500 µm. On réticule le polymère par évaporation des solvants en étuve ventilée pendant 10 min à 85°C. On applique le release ( = feuille plastique enduite de silicone que l'on décolle et que l'on jette avant application) Scotchpak® 1022 (3M) sur la face adhésive du film de polymère. On découpe le film obtenu en patches de 2,5 cm de diamètre (soit 4,909 cm²) pour les tests en cellules de Franz et en patches de 3,5 cm de diamètre (sott 9,621 cm²) pour les tests en dissolutest. On pèse les patches obtenus. On calcule la masse matricielle enduite par patch en soustrayant à la pesée précédente les masses du backing et du release (soit 18 mg/cm²). On calcule la masse matricielle enduite rapportée à 25 cm². On calcule le dosage des patches en multipliant la masse matricielle enduite par le pourcentage de principe actif dans la formulation.

Les patches répondent à la formule suivante :

| Constituants en % de la masse totale | |
|---|---|
| Lauroglycol® (Gattefossé) | 11,5 |
| Propylène glycol (Sigma) | 1,4 |
| Acide caprylique (Sigma) | 1,4 |
| Duro-Tak® 387-2353 (N^{al} Starch & Chemical) | 81,0 |
| Béfloxatone | 4,7 |

Résultats du contrôle des patches (rapportés à 25 cm²)

| Mesures | |
|---|---|
| Masse matricielle enduite (mg) | 334,3 |
| Teneur en béfloxatone dans la préparation (%) | 4,7 |
| Dosage réel (mg) | 15,71 |
| Epaisseur enduite (µm) | 500 |
| Epaisseur sèche (µm) | 115 |

Les différents termes utilisés ci-dessus se définissent comme suit :
- Masse matricielle enduite : masse calculée en soustrayant au poids d'un cm² de patch réticulé le poids correspondant à un cm² de backing et d'un cm² de release (soit 18 mg/cm² au total), et rapportée à la surface du patch (25 cm²).
- Dosage réel : produit de la teneur en béfloxatone dans le mélange initial de formulation par la masse matricielle enduite, et rapportée à la surface du patch.
- Epaisseur enduite : épaisseur d'enduction de la matrice avant la réticulation, donnée par la hauteur de réglage du filmographe.
- Epaisseur sèche : épaisseur d'enduction de la matrice après évaporation des solvants, mesurée par une montre de mesure d'épaisseur.

## Revendications

1. Composition pharmaceutique pour administration transdermique de la béfloxatone, caractérisée en qu'elle comprend de la béfloxatone et au moins un promoteur d'absorption.

2. Composition pharmaceutique selon la revendication 1, **caractérisée en ce que** les promoteurs d'absorption peuvent être choisis parmi : (i) les alcools comprenant de 2 à 36 atomes de carbone, estérifiés ou non par des acides organiques comprenant de 1 à 6 atomes de carbone, (ii) les acides gras comprenant de 5 à 30 atomes de carbone, estérifiés ou non par des alcools comprenant de 1 à 6 atomes de carbone, (iii) les sels alcalins et alcalino-terreux d'acides gras comprenant de 5 à 30 atomes de carbone ou bien un mélange de ces promoteurs d'absorption.

3. Composition pharmaceutique selon la revendication 2, **caractérisée en ce que** le promoteur d'absorption comprend un alcool choisi parmi l'isopropanol, les alcools gras de formule R₁OH, où R₁ représente un radical alkyle linéaire ou ramifié, saturé ou insaturé, comprenant 6 à 30 atomes de carbone, l'alcool benzylique ou le propylène glycol.

4. Composition pharmaceutique selon la revendication 2 ou 3, **caractérisée en ce que** le promoteur d'absorption comprend un acide gras choisi parmi ceux de formule R₂COOH, où R₂ représente un radical alkyle linéaire ou ramifié, saturé ou insaturé comprenant 6 à 30 atomes de carbone, ces acides gras pouvant former des esters avec les alcools méthyliques et éthyliques ou avec le glycérol sous forme de mono, di ou triesters.

5. Composition pharmaceutique selon l'une quelconque des revendications 2 à 4, **caractérisée en ce que** le promoteur d'absorption comprend un sel alcalin ou alcalino-terreux d'acide gras comprenant de 5 à 30 atomes de carbone choisi parmi les sels de sodium, de potassium ou de calcium de ces acides gras.

6. Composition pharmaceutique selon l'une quelconque des revendications 1 à 5. **caractérisée en ce qu'**elle peut prendre la forme de gel, de pommade ou d'émulsion pour une application locale, d'un timbre ou patch transdermique ou d'un film déposé par un spray.

7. Composition pharmaceutique selon l'une quelconque des revendication 1 à 6, **caractérisée en ce qu'**elle prend la forme d'un film transdermique.

8. Composition pharmaceutique selon la revendication 7, **caractérisée en ce qu'**elle prend la forme d'un patch transdermique ou d'une solution pour spray.

9. Composition pharmaceutique sous forme de patch transdermique selon la revendication 8, **caractérisé en ce que** ce dernier comprend un support passif choisi parmi un film métallique, un tissu ou un réseau non tissé de fibres naturelles ou artificielles ou un film polymérique ou bien un constituant actif du système choisi parmi des films ou des matrices polymériques ou un adhésif choisi parmi le caoutchouc naturel ou synthétique, le polyisobutylène, les polyacrylates ou les polyvinyléthers.

10. Composition pharmaceutique sous forme de patch transdermique selon la revendication 9, **caractérisé en ce que** le patch transdermique comprend un film polymérique choisi parmi le polyéthylène, le polypropylène, le polytétrafluoro-éthylène, un polymère cellulosique, acrylique ou vinylique, le silicone ou encore les acrylonitriles.

11. Composition pharmaceutique sous forme d'une solution pour spray selon la revendication 8, **caractérisé en ce que** ce dernier est constitué par une solution comprenant outre (i) la béfloxatone, (ii) un à trois promoteurs d'absorption, (iii) un polymère ou copolymère formant un film souple après évaporation du solvant et (iv) un solvant

12. Composition pharmaceutique sous forme d'une solution pour spray selon la revendication 11, **caractérisé en ce que** le polymère ou copolymère est un polymère ou copolymère qui peut être choisi parmi l'éthylcellulose, l'acétate butyrate de cellulose, l'acétate propionate de cellulose ou une hydroxypropylméthylcellulose greffée ou non ou bien encore un copolymère vinylpyrrolidone / acétate de vinyle.

13. Composition pharmaceutique sous forme d'une solution pour spray selon la revendication 11 ou 12, **caractérisé en ce que** le solvant est choisi parmi l'éthanol, l'isopropanol, l'acétate d'éthyle, l'acétone, le diéthyléther, ou encore un mélange de ces derniers.

14. Composition pharmaceutique selon l'une quelconque des revendications 1 à 13, **caractérisée en ce qu'**elle comprend de 1 à 20% de béfloxatone.

15. Composition pharmaceutique selon l'une quelconque des revendications 1 à 14. **caractérisée en ce que** la teneur totale en promoteur d'absorption par rapport à la quantité totale en béfloxatone varie de 5 à 40%.

## Patentansprüche

1. Pharmazeutische Zubereitung für die transdermale Verabreichung von Befloxaton, **dadurch gekennzeichnet, daß** sie Befloxaton und mindestens einen Absorptions-Promotor umfaßt.

2. Pharmazeutische Zubereitung nach Anspruch 1, **dadurch gekennzeichnet, daß** die Absorptions-Promotoren ausgewählt sein können aus: (i) Alkoholen mit 2 bis 36 Kohlenstoffatomen, die gegebenenfalls mit organischen Säuren mit 1 bis 6 Kohlenstoffatomen verestert sind, (ii) Fettsäuren mit 5 bis 30 Kohlenstoffatomen, die gegebenenfalls mit Alkoholen mit 1 bis 6 Kohlenstoffatomen verestert sind. (iii) Alkali- und Erdalkali-Salzen von Fettsäuren mit 5 bis 30 Kohlenstoffatomen oder einer Mischung dieser Absorptions-Promotoren.

3. Pharmazeutische Zubereitung nach Anspruch 2, **dadurch gekennzeichnet, daß** der Absorptions-Promotor einen Alkohol umfaßt, ausgewählt aus Isopropanol, Fettalkoholen der Formel R₁OH, worin R₁ einen geradkettigen oder verzweigten, gesättigten oder ungesättigten Alkylrest mit 6 bis 30 Kohlenstoffatomen darstellt, Benzylalkohol oder Propylenglykol.

4. Pharmazeutische Zubereitung nach Anspruch 2 oder 3, **dadurch gekennzeichnet, daß** der Absorptions-Promotor eine Fettsäure umfaßt, ausgewählt aus solchen der Formel R₂COOH, worin R₂ einen geradkettigen oder verzweigten, gesättigten oder ungesättigten Alkylrest mit 6 bis 30 Kohlenstoffatomen darstellt, wobei diese Fettsäuren Ester bilden können mit Methylalkohol und Ethylalkohol oder mit Glycerol in Form der Mono-, Di- oder Triester.

5. Pharmazeutische Zubereitung nach einem der Ansprüche 2 bis 4, **dadurch gekennzeichnet, daß** der Absorptions-Promotor ein Alkalisalz oder Erdalkalisalz einer Fettsäure mit 5 bis 30 Kohlenstoffatomen, ausgewählt aus den Natrium-, Kalium- oder Calcium-Salzen dieser Fettsäuren, umfaßt.

6. Pharmazeutische Zubereitung nach einem der Ansprüche 1 bis 5, **dadurch gekennzeichnet, daß** sie in Form eines Gels, einer Salbe oder einer Emulsion für die lokale Anwendung, eines transdermalen Klebepräparats oder Pflasters oder eines mit Hilfe eines Sprühprodukts aufgebrachten Films vorliegt.

7. Pharmazeutische Zubereitung nach einem der Ansprüche 1 bis 6, **dadurch gekennzeichnet, daß** sie in Form eines transdermalen Films vorliegt.

8. Pharmazeutische Zubereitung nach Anspruch 7, **dadurch gekennzeichnet, daß** sie in Form eines transdermalen Pflasters oder einer zu versprühenden Lösung vorliegt.

9. Pharmazeutische Zubereitung in Form eines transdermalen Pflasters nach Anspruch 8, **dadurch gekennzeichnet, daß** dieses einen passiven Träger umfaßt, ausgewählt aus einem Metallfilm, einem Gewebe oder einem nicht gewebtem Netz aus natürlichen oder künstlichen Fasern oder einem Polymerfilm oder einem aktiven Bestandteil des Systems, ausgewählt aus polymeren Filmen oder Matrices oder einem Klebstoff, ausgewählt aus natürlichem oder synthetischem Kautschuk, Polyisobutylen, Polyacrylaten oder Polyvinylethern.

10. Pharmazeutische Zubereitung in Form eines transdermalen Pflasters nach Anspruch 9, **dadurch gekennzeichnet, daß** das transdermale Pflaster einen Polymerfilm umfaßt, ausgewählt aus Polyethylen, Polypropylen, Polytetrafluorethylen, einem Cellulose-, Acryl- oder Vinyl-Polymer, Silicon oder Acrylnitrilen.

11. Pharmazeutische Zubereitung in Form einer zu versprühenden Lösung nach Anspruch 8, **dadurch gekennzeichnet, daß** diese durch eine Lösung gebildet ist, die neben (i) Befloxaton, (ii) ein bis drei Absorptions-Promotoren, (iii) ein Polymer oder Copolymer, welches nach dem Verdampfen des Lösungsmittels einen schmiegsamen Film ergibt, und (iv) ein Lösungsmittel, umfaßt.

12. Pharmazeutische Zubereitung in Form einer zu versprühenden Lösung nach Anspruch 11, **dadurch gekennzeichnet, daß** das Polymer oder Copolymer ein Polymer oder Copolymer ist, welches aus Ethylcellulose. Cellulose-acetat-butyrat, Cellulose-acetat-propionat oder einer gegebenenfalls gepfropften Hydroxypropylmethylcellulose oder einem Vinylpyrrolidon/Vinylacetat-Copolymer ausgewählt sein kann.

13. Pharmazeutische Zubereitung in Form einer zu versprühenden Lösung nach Anspruch 11 oder 12, **dadurch gekennzeichnet, daß** das Lösungsmittel ausgewählt ist aus Ethanol, Isopropanol, Ethylacetat, Aceton, Diethylether oder einer Mischung dieser Lösungsmittel.

14. Pharmazeutische Zubereitung nach einem der Ansprüche 1 bis 13, **dadurch gekennzeichnet, daß** sie 1% bis 20% Befloxaton enthält.

15. Pharmazeutische Zubereitung nach einem der Ansprüche 1 bis 14, **dadurch gekennzeichnet, daß** der Gesamtgehalt an Absorptions-Promotor bezogen auf die Gesamtmenge an Befloxaton zwischen 5% und 40% liegt.

## Claims

1. Pharmaceutical composition for transdermal administration of befloxatone, **characterized in that** it comprises befloxatone and at least one absorption promoter.

2. Pharmaceutical composition according to Claim 1, **characterized in that** the absorption promoters may be chosen from: (i) alcohols comprising from 2 to 36 carbon atoms, optionally esterified with organic acids comprising from 1 to 6 carbon atoms, (ii) fatty acids comprising from 5 to 30 carbon atoms, optionally esterified with alcohols comprising from 1 to 6 carbon atoms, (iii) alkali and alkaline-earth metal salts of fatty acids comprising from 5 to 30 carbon atoms or alternatively a mixture of these absorption promoters.

3. Pharmaceutical composition according to Claim 2, **characterized in that** the absorption promoter comprises an alcohol chosen from isopropanol, fatty alcohols of formula R₁OH, where R₁ represents a saturated or unsaturated, linear or branched alkyl radical comprising 6 to 30 carbon atoms, benzyl alcohol or propylene glycol.

4. Pharmaceutical composition according to Claim 2 or 3, **characterized in that** the absorption promoter comprises a fatty acid chosen from those of formula R₂COOH, where R₂ represents a saturated or unsaturated, linear or branched alkyl radical comprising 6 to 30 carbon atoms, it being possible for these fatty acids to form esters in the form of mono-, di- or triesters with methyl and ethyl alcohols or with glycerol.

5. Pharmaceutical composition according to any one of Claims 2 to 4, **characterized in that** the absorption promoter comprises an alkali or alkaline-earth metal salt of fatty acid comprising from 5 to 30 carbon atoms chosen from the sodium, potassium or calcium salts of these fatty acids.

6. Pharmaceutical composition according to any one of Claims 1 to 5, **characterized in that** it may take the form of a gel, of an ointment or of an emulsion for local administration, of a transdermal patch or of a film deposited by a spray.

7. Pharmaceutical composition according to any one of Claims 1 to 6, **characterized in that** it takes the form of a transdermal film.

8. Pharmaceutical composition according to Claim 7, **characterized in that** it takes the form of a transdermal patch or of a spray solution.

9. Pharmaceutical composition in the form of a transdermal patch according to Claim 8, **characterized in that** the latter comprises a passive support chosen from a metal film, a nonwoven fabric or a nonwoven network of natural or artificial fibres or a polymeric film or an active constituent of the system chosen from polymeric films or polymeric matrices or an adhesive chosen from natural or synthetic rubber, polyisobutylene, polyacrylates or polyvinyl ethers.

10. Pharmaceutical composition in the form of a transdermal patch according to Claim 9, **characterized in that** the transdermal patch comprises a polymeric film chosen from polyethylene, polypropylene, polytetrafluoroethylene, a cellulosic, acrylic or vinyl polymer, silicone or alternatively acrylonitriles.

11. Pharmaceutical composition in the form of a spray solution according to Claim 8, **characterized in that** the latter consists of a solution comprising in addition (i) befloxatone, (ii) one to three absorption promoters, (iii) a polymer or copolymer forming a flexible film after evaporation of the solvent and (iv) a solvent.

12. Pharmaceutical composition in the form of a spray solution according to Claim 11, **characterized in that** the polymer or copolymer is a polymer or copolymer which may be chosen from ethyl cellulose, cellulose acetate butyrate, cellulose acetate propionate or a hydroxypropylmethylcellulose grafted or otherwise, or alternatively a vinylpyrrolidone vinyl acetate copolymer.

13. Pharmaceutical composition in the form of a spray solution according to Claim 11 or 12, **characterized in that** the solvent is chosen from ethanol, isopropanol, ethyl acetate, acetone, diethyl ether, or alternatively a mixture thereof.

14. Pharmaceutical composition according to any one of Claims 1 to 13, **characterized in that** it comprises from 1 to 20% of befloxatone.

15. Pharmaceutical composition according to any one of Claims 1 to 14, **characterized in that** the total content of absorption promoter relative to the total quantity of befloxatone varies from 5 to 40%.
